# EUROPEAN PATENT SPECIFICATION

(11) **EP 1 368 044 B1**
(45) Date of publication and mention of the grant of the patent: **18.03.2009**
(21) Application number: 02715607.4
(22) Date of filing: 17.01.2002
(51) Int. Cl.: A61K 35/74, A61P 37/02, A61P 31/18

(54) **IMMUNOMODULATOR FOR THE MANAGEMENT OF HUMAN IMMUNODEFICIENCY VIRUS (HIV) DISEASE/INFECTION**
IMMUNOMODULATOR ZUR BEKÄMPFUNG VON DURCH HUMANEN IMMUNSCHWÄCHEVIRUS (HIV) VERMITTELTE KRANKHEITEN/INFEKTIONEN
IMMUNOMODULATEUR DE GESTION THERAPEUTIQUE DE L'INFECTION/MALADIE DE L'IMMUNODEFICIENCE HUMAINE (VIH)

(43) Date of publication of application: 10.12.2003
(73) Proprietor: Bakulesh, Mafatlal Khamar, Ahmedabad 380-006, Gujarat (IN); Indravadan, Ambalal Modi, Cadila Pharmaceuticals Limited, Ahmedabad 380 009, Gujarat (IN)
(72) Inventor: Bakulesh, Mafatlal Khamar, Ahmedabad 380-006, Gujarat (IN); Indravadan, Ambalal Modi, Cadila Pharmaceuticals Limited, Ahmedabad 380 009, Gujarat (IN)
(74) Representative: Towler, Philip Dean
(86) International application number: PCT/IB2002/000098
(87) International publication number: WO 2002/056898

(56) References cited:
- WO-A-94/06466
- GB-A- 2 236 480
- GULERIA I ET AL: "In vivo depletion of CD4 and CD8 T lymphocytes impairs Mycobacterium w vaccine-induced protection against M. tuberculosis in mice." MEDICAL MICROBIOLOGY AND IMMUNOLOGY. GERMANY JUL 1993, vol. 182, no. 3, July 1993 (1993-07), pages 129-135, XP001087510 ISSN: 0300-8584
- REDDI P P ET AL: "Molecular definition of unique species status of Mycobacterium w;a candidate leprosy vaccine strain." INTERNATIONAL JOURNAL OF LEPROSY AND OTHER MYCOBACTERIAL DISEASES, (1994 JUN) 62 (2) 229-36. , XP008014392
- TALWAR G P: "An immunotherapeutic vaccine for multibacillary leprosy." INTERNATIONAL REVIEWS OF IMMUNOLOGY, (1999) 18 (3) 229-49. REF: 34 , XP008014406
- JOHNSON D ET AL: "Randomised trial of intradermal Mycobacterium vaccae or intradermal hepatitis B immunisation in children with HIV infection" VACCINE, BUTTERWORTH SCIENTIFIC. GUILDFORD, GB, vol. 17, no. 20-21, 4 June 1999 (1999-06-04), pages 2583-2587, XP004169669 ISSN: 0264-410X

## Description

This invention relates to immunomodulator for the management of human immunodeficiency virus (hiv) disease/infection.

### BACKGROUND OF THE INVENTION

Human immunodeficiency virus (HIV) was first isolated in 1983. The causative agent for AIDS is known to be a virus of the retrovirus family called HIV (human immunodeficiency virus). Infection with HIV does not, however, immediately give rise to overt symptoms of AIDS. Three to six weeks following primary HIV infection more than 50% of individuals develop acute HIV syndrome, which is self limiting. Clinical findings seen during this period include fever, pharyngitis, lymphadenopathy, headache, arthralgia, myalgia, malaise, lethargy, nausea, vomiting, diarrhoea, skin rash, mucocutaneous ulceration, meningitis, encephalitis, neuropathy etc. The only indication of exposure to the virus may be the presence of antibodies thereto in the blood of an infected subject who is then described as 'HIV positive'. The infection may lie dormant; giving rise to no obvious symptoms, and the incubation period prior to development of AIDS may vary from several months to decades. Development of AIDS itself may be preceded by the AIDS-related complex (ARC), which is characterized by unexplained fever, weight loss, chronic cough or diarrhea. The development of AIDS and/or ARC is dependent on breakdown of immune system. The reasons for the variable period between infection with the virus and breakdown of the immune system in an infected individual are poorly understood. Factors at present unknown may trigger proliferation of the virus with consequential disruption of the immune system. The victims of the disease are then subject to various infections and malignancies, which, unchecked by the disabled immune system, lead to death. Thus HIV is characterized by the "acute HIV syndrome" followed by "asymptomatic stage" with clinical latency. Symptomatic stage sets in later with breakdown of immune system, which ultimately leads to the death of the individual infected with HIV.

Though the disease is caused by virus, the morbidity and mortality associated with disease is due to breakdown of immune system. The breakdown of immune system is characterized by decreased CD4 ⁺ T lympocyte count. Because of this reason 1993 revised classification system for HIV infection is based on CD4⁺ T lymphocyte counts. HIV disease is empirically divided based on CD₄ count which is a measure of immunodeficiency.
a) Early stage CD₄ + T cell count more than 500
b) Intermediate stage CD₄ + T cell count 200 to 500.
c) Advanced stage CD₄ + T cell count less than 200.

Individuals with nonprogressive HIV disease are found to have steady CD₄ counts. They are also observed to have strong immune response against the virus.

There is evidence that in HIV infection, there is a dramatic loss of CD4 T-cells, which results in very rapid development of overt symptoms of AIDS. Most AIDs defining opportunistic infections and true malignancies occur in advanced stage of disease where in CD₄ count is less the 200 cells/µL.

### CD4 count and HIV

Though HIV is a viral infection, viral load can be determined by reasonable accuracy, CD4 count (a measure of immune status) plays major role in management of HIV due to following reasons.
1. Morbidity and Mortality in HIV infected individuals is due to opportunistic infections. These opportunistic infections define onset of AIDS in HIV +ve individuals.The risk of opportunistic disease increases markedly when CD4+ cell count declines to less than 200 cells/mm³.
2. CD4 count provides estimate of degree of existing immunodeficiency. Immune deficiency is responsible for HIV +ve individuals getting converted to AIDS.
3. The initiation of antiretroviral therapy is also dependent on CD4 count.
4. Outcome of antiretroviral therapy is also dependent on CD4 count. Higher survival are associated with higher initial CD4 count.
5. Risk of progression to AIDs defining illness is associated with declining CD4 count. The risk is lower with higher CD4 count.
6. Likelyhood of developing AIDs within 3 years is significantly higher when CD4 count is low (less than 200 CD4 + T cells) compared to high CD4 + T cell count. For viral load of greater than 55k as per RT-PCR the risk is 32.6% if CD4 + T cell count is more than 500 cells/mm3 compared to 85.5% for individuals with CD4 count of less than 200 cells/mm³.
7. Similarly for viral load of 20k-55k (RT-PCR) the risk of developing AIDs is 9.5% when CD4 count is more than 750 cells/mm3 compared to 40.1% when CD4 count is less than 350 cells/mm3.

Goals of therapy
- Maximal and durable suppression of viral load.
- Restoration and/or preservation of immunologic function.
- Improvement of quality of life.
- Reduction in HIV related morbidity and mortality.

The method to treat HIV includes various therapeutic options. The options include management of symptoms and infections manifesting in HIV infected individuals at various stages of the disease. The antiretroviral drugs are used to keep the HIV infection (viral load) in control. They keep the viral load in control. The early antiretroviral drugs like azothymidine delayed progression of disease and had no significant effect on CD₄ count. Protease inhibitors like indinavir, ritonavir which are introduced recently do improve CD₄ count while reducing viral load. All the drugs (antiretroviral) have their own side effects. The resistance to drugs is also noted. Thus there is need to provide alternate mechanism of treating HIV.

Since CD₄ count is important in maintaining immunity of individual and decreased CD₄ counts are associated with morbidity and mortality in HIV infection attempts are made to improve immunity for management of HIV. Various efforts have been done towards this end. This has resulted in introduction of immune modifying therapies with or without antiretroviral drugs. They comprise of antigens, cytokines organisms etc.

It has surprisingly been found during the course of research by us that formulations of 'Mycobacterium W' (M_{w}) with or without antigenic and/or immunomodulatory material derived from (M_{w}) is effective for management of Human Immunodeficiency Virus (HIV) disease/infection.

### Prior Art

M. vaccae is apparently unique among known mycobacterial species in that heat-killed preparations retain its properties for the use as vaccine and immunotherapeutic. For example, M. bovis-BCG vaccines, used for vaccination against tuberculosis, employ live strains. Heat-killed M. bovis BCG and M. tuberculosis have no protective properties when employed in vaccines. A number of compounds have been isolated from a range of mycobacterial species, which have adjuvant properties. The effect of such adjuvants is essentially to stimulate a particular immune response mechanism against an antigen from another species.

In US Patent No. 6,001,361 the invention is related to compounds and methods for the treatment of mycobacterial infections including Mycobacterium tuberculosis and Mycobacterium avium. The invention is further related to compounds that function as non-specific immune response amplifiers, and the use of such non-specific immune response amplifiers as adjuvants in vaccination or immunotherapy against infectious disease, and in certain treatments for immune disorders and cancer..

U.S. Pat. No. 4,716,038 discloses diagnosis of, vaccination against and treatment of autoimmune diseases of various types, including arthritic diseases, by administering mycobacteria, including M. vaccae.

US 6210684 describes method for delaying the onset of AIDS using killed M. Vaccae. Onset of AIDS is related to decrease in CD₄ count is not disclosed in the patent. Published studies shows that killed M. Vaccae has no effect on CD₄ count in HIV positive individuals.

International Patent Publication WO 91/01751 discloses the use of antigenic and/or immunoregulatory material from M. vaccae as an immunoprophylactic to delay and/or prevent the onset of AIDS.

International Patent Publication WO 94/06466 discloses the use of antigenic and/or immunoregulatory material derived from M. vaccae for therapy of HIV infection, with or without AIDS and with or without associated tuberculosis.

US Patent No.6,001,361 discloses an invention that relates generally to the treatment by vaccination or immunotherapy of skin disorders such as psoriasis, atopic dermatis, allergic contact dermatitis, alopecia areata, and the skin cancers basal cell carcinoma, squamous cell carcinoma and melanoma. In particular, the invention is related to the use of compounds, which are present in or have been derived from Macobacterium vaccae (M. vaccae) or from the culture filtrate of M. vaccae.

U.S. Pat. No. 5,599,545 discloses the use of mycobacteria, especially whole, inactivated M. vaccae, as an adjuvant for administration with antigens, which are not endogenous to M. vaccae. This publication theories that the beneficial effect as an adjuvant may be due to heat shock protein 65 (hsp 65).

International Patent Publication WO 92/08484 discloses the use of antigenic and/or immunoregulatory material derived from M. vaccae for the treatment of uveitis.

International Patent Publication WO 93/16727 discloses the use of antigenic and/or immunoregulatory material derived from M. vaccae for the treatment of mental diseases associated with an autoimmune reaction initiated by an infection.

International Patent Publication WO 95/26742 discloses the use of antigenic and/or immunoregulatory material derived from M. vaccae for delaying or preventing the growth or spread of tumors.

Vacce is not associated with change in CD₄ count or viral load in HIV positive individuals. It does not provide any relief in HIV +ve individual.
However inspite of various patents issued in relation to mycobacterium vaccae, it fails to provide any significant change in CD₄ count as well as viral load in individuals who are HIV positive.

Similarly attempts have been made to improve CD₄ count using immunomodulator from various sources.

Remune is a HIV-1 specific immunogen in incomplete Freund's adjuvant. It includes inactivated HIV-1 from which gp 120 is depleted. It is found to be safe with immunogenic potential in persons infected with HIV. In initial studies it was found to improve CD4 - cell count in asymptomatic HIV cohort not taking antiretroviral agents. It is given intramuscularly as an injection into the triceps muscles. The recent study by Sukeepaisarncharoen w et at suggests that remune therapy is associated with stabilization of CD 4 -cell counts. It is also suggests that it may be of value in participants with higher CD4+ T cell count.

One such immunomodulator consists of mixture of antigens of inactivated bacteria with antigens of influenza virus (poly antigenic immunomodulator). It has not been possible to achieve improvement in CD₄ count using it.

SB-73 is another immunomodulator made up of substance produced by pencillium P(PB-73 strain). In a small study it is found to improve CD₄ count in majority(10/14) of individuals, infected with HIV when given intramuscularly in a dose of 5 mgm.

Reticulose, a peptide-nucleic acid is another immunomodulator found to be useful in improving CD₄ count in HIV infected individuals when given subcutaneously.

It was given as two 1 ml subcutaneous(SC) injections per day for two weeks followed by 1 ml SC perday every other week for a total 60 days (30 days total treatment). It resulted in a significant improvement in CD₄ count in absence of any other antiretroviral therapy.

Thymosin α₁ is a 28-aminoacid peptide. It was evaluated for its efficacy to improve CD₄ along with interleukin-2. It was found to have no significant effect. Grenulocyte colong stimulating factor(Filgrastim) has also been evaluated to improve immune response in HIV without much success.

OKT3, a CD₃ monoclonal antibody, has been successfully used in management of HIV along with antiretroviral and Interleukin-2 in three patients.

Of various cytokines used in management of HIV, Interleukin -2 (IL-2) is extensively studied and used. It is used as injection to be administered intravenously or subcutaneously. It is found to improve CD₄ count significantly when used alone or along with antiretroviral drugs. It is given as intermittent therapy the side effects seen are sometimes intolerable. They are seen only during period of active administration. The improvement seen in CD₄ count is found to be stable.

Other cytokines used in management of HIV includes IL-12 and IL-15.

US 5759992 provides low molecular weight glycopeptide with a molecular weight of 919.2 dalton which is derived from supernatant of disrupted cells maintaining temprature of 4° C through out the process. This is obtained from bacteria which includes E coli and Mycobacterium. It is found to improve CD₄ count in normal mice. It is not evaluated in HIV +ve animals.

US patent 5871732 describes methods for preventing or treating AIDS, AIDS related complex and human immunodeficiency virus infection by anti-CD₄ antibody homologs to DNA sequences of encoding such homologs.

Mycobacterium w is a non-pathogenic, cultivable, atypical mycobacterium, with biochemical properties and fast growth characteristics resembling those belonging to Runyons group IV class of Mycobacteria in its metabolic and growth properties but is not indentical to those strains currently listed in this group. It is therefore thought that (M_{w}) is an entirely new strain. The species identity of M_{w} has been defined by polymerase chain reaction DNA sequence determination.

It has been found to share antigens with Mycobacterium leprae and Mycobacterium tuberculosis. It is found to provide prophylaxis against leprosy in humans by converting lepromin negative individuals to lepromin positivity. It is also found to provide prophylaxis against tuberculosis in animals. In leprosy it is also found to reduce duration of therapy for bacterial killing, clearance as well as clinical cure when used along with multi drug therapy.

### Reference

1. Immunological parameters modified in HIV disease by the macrophage activity immunomodulator WF 10 (a phase II pathogenesis study) Herndier B; Lull R Ah Ching O; Broz M; Kuehne FW; Kahn J Int Conf AIDS. 1998;12:347-8
2. Changes in viral expression and cytokine profile induced by a polyantigenic immunomodulator in HIV-infected peripheral blood mononuclear cells. Rios Z; Rios EO; Garcia MI; De Leon C; Guzman LM; Rodriguez W; Romero D; Hunter R.Cell Mol Biol (Noisy-le-grand). 1995;41 Suppl 1:s93-101.
3. Controlled clinical trial of reticulose, a peptide-nucleic acid with Imunomodulator activity, in patients with HIV infection. Levett PN; Roach TC; Hirschman SZ; Broome H; Fraser HS Abstr Gen Meet Am Soc Microbiol. 1998 May 17-21; 98:53
4. Survival and quality of life in HIV-positive patients treated with poly antigenic immunomodulator. Colon JI; Encarnacion G; Ortiz Santini MR; Rodriguez Malave MT; Santiago Delpin EA; Marchand AM P R Health Sci J. 1997 Mar; 16(1):9-14.
5. SB-73 immunomodulator: phase II clinical trial on HIV infection. Bellucci S; Abreu W; Bertazzoli R; Tomita F; Lourenco C; Machodo L; Silva O Int Conf AIDS. 1992 Jul 19-24;8(3):54
3. Interleukin-15 Triggers Activation and Growth of the CD8 T-Cell Pool in Extravaxcular Tissues of Patients With Acquired Immunodificiency Syndrome. Agostini C, Trentin L, Sancetta R, Facco M, Tassinari C, Cerutti A, Bortolin M, Milani A, Siviero M, Zambello R, Semenzato G. Blood, Vol 90, No 3 (August 1), 1997:pp 1115-1123
4. Ethylene Glycol-Modified Interleukin-2 and Thymosin α1 in Human immunodefiency Virus Type 1 Infection Journal of Infecious Disease, 1996;73;1005-8
5. OKT3 and IL-2 treatment for purging of the latent HIV-1 reservoir in vivo results in selective long-lasting CD4+ T cell depletion. van Praag RM, Prins JM, Roos MT, Schellekens PT, Ten Berge IJ, Yong SL, Schuitemaker H, Eerenberg AJ, Jurriaans S, de Wolf F, Fox CH, Goudsmit J, Miedema F, Lange JM J Clin Immunol 2001 May;21(3):218-26
6. How effective are complementary therapies for HIV and AIDs?-A systemic review. Ozsoy M, Ernst E. Int J STD AIDS 1999 Oct; 10 (10) : 629-35
7. Mechanism of HIV persistence: implications for vaccines and therapy. Bremermann HJ J Acquir Immune Defic Syndr Hum Retrovirol 1995 Aug 15;9(5):459-83.
8. Both serum HIV type 1 RNA levels and CD4 lymphocyte counts predict clinical outcome in HIV type 1- infected subjects with 200 to 500 CD4 + cells per cubic millimeter. AIDs clinical trial group study 175 virology study team. Kim S, Hughes MD, Hammer SM, Jackson JB, DeGruttola V, Katzenstein DA. AIDs Res Hum Retroviruses 2000 May 1;16(7): 645-53.
9. Do HIV type 1 RNA levels provide additional prognostic value to CD4 (+) T lymphocyte counts in patients with advanced HIV type 1 infection? AIDs Res Hum Retroviruses 2001 Aug 10;17(12):1099-105.
10. Guidelines for the Use of Antiretroviral Agents in HIV-Infected Adults and Adolescents. August 13, 2001 (This guidelines were developed by the Panel on Clinical Practices for Treatment of HIV Infection convened by the Department of Health and Human Services (DHHS) and the Henry J. Kaiser Family Foundation. Leadership of the Panel consists of Anthony S. Fauci, National Institutes of Health, Bethesda, MD (co-chair); Eric P. Goosby, DHHS, Washington, DC, (co-convener); and Jennifer Kates, Henry J. Kaiser Foundatin, San Francisco, CA, (co-convener).
11. A controlled Trial of two nucleoside analogues plus indinavir in persons with human immunodeficiency virus infection and CD4 cell counts of 200 per cubic millimeter or less. Arduino JM, Fischl MA, Stanley K, Collier AC, Spiegelman D. The New Eng. Journal of Med. 1997 Sep.337(11):725-733.
12. Treatment with Indinavir, Zidovudine, and Lamivudine in adults with human immunodeficiency virus infection and prior antiretroviral therapy. scott m.hammer,m.d.,kathleen e.squires,m.d.,michael d.hughes,ph.d.,janet m.grimes,m.s.lisam.emeter,m.d.judith s.currier.,m.d.,josephj.eron,jr.,m.d.,judith e.feinberg,m.d.,henry h.balfour,jr.,m.d.,lawrence r.eyton,m.d.,jeffrey a.chodakewitz,m.d., et al. The New Eng. Journal of Med. 1997 September 337(11):734-739.
13. Ramdomised placebo-controlled trial of ritonavir in advanced HIV-1 disease Yazdanpanah Y, Chene G, Losina E, Goldie SJ, Merchadou LD, Alfandari S, Seage GR 3rd, Sullivan L, Marimoutou C, Paltiel AD, Salamon R, Mouton Y, Freedberg KA. Int J Epidemiol. 2001 Aug;30(4):864-71.
22. Randomised trial of intrademial Mycobacterium vaccae or intradermal hepatitis B immunisation in children with HIV infection. Johnson D, Waddell RD, Pelton SI, Jaeger AS, Modlin JF, Yogev R, Morin P, Arbeit RD, von Reyn CF. Vaccine. 1999 Jun 4; 17(20-21):2583-7.
23. Immunization of HIV-infected adults with a three-dose series of inactivated Mycobacterium vaccae. Marsh BJ, Fordham von Reyn C, Arbeit RD, Morin P. Am J Med Sci. 1997 Jun;313(6):377-83.
24. Safety and immunogenicity of a five-dose series of inactivated Mycobacterium vaccae vaccination for the prevention of HIV-associated tuberculosis. Waddell RD, Chintu C, Lein AD, Zumla A, Karagas MR, Baboo KS, Habbema JD, Tosteson AN, Morin P, Tvaroha S, Arbeit RD, Mwinga A, von Reyn CF. Clin Infect Dis. 2000 Jun;30 Suppl 3:S309-15.

### Summary of the invention

According to present invention, immunomodulator made from 'Mycobacterium w' (M_{w}) is found to be useful in, the management of HIV infection. We have now found that the same therapeutic agent not only delays development of AIDS in patients infected by HIV, but also is capable of causing regression, or even removal, of overt symptoms of AIDS even in patients where the disease is far advanced. These effects have been found in patients suffering also from tuberculosis. These effects are also seen in patients who are suffering from HIV infection with or without AIDS and without associated tuberculosis. The immunomodulator as per present invention is also found useful in relieving symptoms of HIV.

Therapeutic agent which may be used in the present invention resembles Mw a non-pathogenic, cultivable, atypical mycobacterium, with biochemical properties and fast growth characteristics resembling those belonging to Runyons group IV class of Mycobacteria in its metabolic and growth properties but is not indentical to those strains currently listed in this group. It is therefore thought that (M_{w}) is an entirely new strain.

The species identity of Mw has been defined by polymerase chain reaction DNA sequence determination and differentiated from thirty other species of mycobacteria. It however differs from those presently listed in this group in on respect or the other. By base sequence analysis of a polymorphic region of pattern analysis, it has been established that M_{w} is a unique species distinct from many other known mycobacterial species examined which are: M. avium, M. intracellulare, M. scrofulaceum, M. kansasii, M. gastri, M. gordonae, M. shimoidei, M. malmoense, M. haemophilum, M. terrae, M. nonchromogenicum, M. triviale, M. marinum, M. flavescens, M. simian, M. szulgai, M. xenopi, M. asciaticum, M. aurum, M. smegmatis, M. vaccae, M. fortuitum subsp fortuitum, M. fortuitum subsp. Peregrinum, M. chelonae subsp. Chelonae, M. chelonae subsp. Abscessus, M. genavense, M. tuberculosis, M. tuberculosis H₃₇Rᵥ, M. paratuberculosis.

The object of the present invention is to provide an immunomodulator Mycobacterium w containing 'Mycobacterium w' (Mw) with or without obtained from Mw for the prophylaxis or therapy of AIDS or AIDS related complex, to a subject exposed to HIV infection or is HIV positive with or without overt symptoms of AIDS.

Yet another object of the invention is to provide immunomodulator derived from mycobacterium w that are useful for the management of HIV infection.
Yet another object of the invention is to provide immunomodulator derived from Mycobacterium w to improve immune function of HIV +ve subjects.

Yet another object of present invention is to provide immunomodulator to improve CD₄ count in HIV infected individuals.
Yet another object of present invention is to provide immunomodulator effective in ameliorating symptoms associated with HIV infection.
Yet another object of present invention is to provide an immunomodulator effective in management of opportunistic infections of associated with HIV infection.
Yet another object to present invention is to provide an immunomodulator useful in improving immune function of HIV +ve subjects in presence or absence of antiretroviral drugs.

Yet another object of the invention is to provide methods for the preparation of a medicament for the treatment of HIV, which results in the amelioration of symptoms of symptomatic stage of the disease.

It is yet another object of the invention to provide for the preparation of a medicament for the treatment of HIV infection that results in improved CD4⁺T cell count.

### DETAILED DESCRIPTION OF THE INVENTION

In accordance with the invention the composition of immunomodulator the method of preparation, HPLC charachteristic its safety and tolerability, methods of use and outcome of treatments are described in following examples. The following are illustrative examples of the present invention and scope of the present invention should not be limited by them.

### Example 1. The immunomodulator compositions:

A. Each dose of 0.1 ml of therapeutic agent contains:

| | |
|---|---|
| Mycobacterium w., (heat killed) | 0.50 × 10⁹ |
| Sodium Chloride I. P. | 0.90% w/v |
| Thiomerosal I. P. | 0.01% w/v |
| (As a Preservative) | |
| Water for injection I. P. | q. s. to 0.1 ml |

B. Each dose of 0.1 ml of therapeutic agent contains

| Extract of Mycobacterium w after sonication from 1×10¹⁰ Mycobacterium w | |
|---|---|
| Sodium Chloride I. P. | 0.90% w/v |
| Thiomerosal I. P. | 0.01 % w/v |
| (As a Preservative) | |
| Water for injection I. P. | q. s. to 0.1 ml |

C. Each dose of 0.1 ml of therapeutic agent contains

| Methanol Extract of 1×10¹⁰ Mycobacterium w | |
|---|---|
| Sodium Chloride I. P. | 0.90% w/v |
| Thiomerosal I. P. | 0.01 % w/v |
| (As a Preservative) | |
| Water for injection I. P. | q. s. to 0.1 ml |

D. Each dose of 0.1 ml of therapeutic agent contains

| Chloroform Extract of 1×10¹⁰ Mycobacterium w | |
|---|---|
| Sodium Chloride I. P. | 0.90% w/v |
| Thiomerosal I. P. | 0.01% w/v |
| (As a Preservative) | |
| Water for injection I. P. | q. s. to 0.1 ml |

E. Each dose of 0.1 ml of therapeutic agent contains

| Acetone Extract of 1×10¹⁰ Mycobacterium w | |
|---|---|
| Sodium Chloride I. P. | 0.90% w/v |
| Thiomerosal I. P. | 0.01 % w/v |
| (As a Preservative) | |
| Water for injection I. P. | q. s. to 0.1 ml |

F. Each dose of 0.1 ml of therapeutic agent contains

| Ethanol Extract of 1×10¹⁰ Mycobacterium w | |
|---|---|
| Sodium Chloride I. P. | 0.90% w/v |
| Thiomerosal I. P. | 0.01% w/v |
| (As a Preservative) | |
| Water for injection I. P. | q. s. to 0.1 ml |

G. Each dose of 0.1 ml of therapeutic agent contains

| Liticase Extract of 1×10¹⁰Mycobacterium w | |
|---|---|
| Sodium Chloride I. P. | 0.90% w/v |
| Thiomerosal I. P. | 0.01% w/v |
| (As a Preservative) | |
| Water for injection I. P. | q. s. to 0.1 ml |

H. Each dose of 0.1 ml of therapeutic agent contains

| Mycobacterium w (heat killed) 0.5x10⁷ | |
|---|---|
| Extract of mycobacterium w obtained 1×10³ Mycobacterium w by disruption, solvent extraction or enzymatic extraction. | |
| Sodium Chloride I. P. | 0.90% w/v |
| Thiomerosal I . P. | 0.01 % w/v |
| (As a Preservative) | |
| Water for injection I. P. | q. s. to 0.1 ml |

### Example 2. The Process of preparing immunomodulator

A. Culturing using of Mycobacterium w.
   i) Preparation of calture medium.
      Mycobacterium w is cultured on solid medium like L J medium or liquid medium like middle brook medium or sauton's liquid medium.
      For better yield middle brook medium is enriched. It can be preferably enriched by addition of glucose, bactotryptone, and BSA. They are used in ratio of 20:30:2 preferably.
      The enrichment medium is added to middle brook medium. It is done preferably in ratio of 15:1 to 25:1 more preperably in ratio of 20:1.
   ii) Bioreactor operation
      a) Preparation of vessel:
         The inner contact parts of the vessel (Joints, mechanical seals, o-ring/gasket grooves, etc.) should be properly cleaned to avoid any contamination. Fill up the vessel with 0.1 N NaOH and leave as such for 24 H to remove pyrogenic materials and other contaminants. The vessel is then cleaned first with acidified water, then wit ordinary water. Finally, the vessel is rinsed with distilled water (3 times) before preparing medium.
      b) Sterilization of bioreactor
         The bioreactor containing 9L distilled water is sterilized with live steam(indirect). Similarly the bioreactor is sterilized once more with Middlebrook medium. The other addition bottles, inlet/outlet air filters etc. are autoclaved (twice) at 121°C for 15 minutes. Before use, these are dried at 50° C oven.
      c) Environmental parameter
         i. Temprature: 37± 0.5° C
         ii. pH : 6.7 to 6.8 initially.
B. Harvesting and concentrating
   It is typically done at the end of 6^{th} day after culturing under aseptic condition. The concentration of cells (palletisation) is done by centrifugation.
C. Washing of cells
   The pallet so obtained is washed minimum three times with normal saline. It can be washed with any other fluid which is preferably isotonic.
D. Adding pharmaceutically acceptable carrier.
   Pyrogen free normal saline is added to pallet. Any other pyrogen free isotonic fluid can be used as a pharmaceutical carrier. The carrier is added in amount so as get to desired concentration of active in final form.
E. Adding preservative
   To keep the product free from other contaminating bacteria for its self life preservative is added. Preferred preservative is thiomesol which is used in final concentration of 0.01 % w/v.
F. Terminal Sterilization
   Terminal sterilization can done by various physical methods like application of heat or ionizing radiation or sterile filtration.
   Heat can be in the form of dry heat or moist heat. It can also be in the form of boiling or pasturisation.
   Ionizing radiation can be ultraviolet or gamma rays or mircrowave or any other form of ionizing radiation.
   It is preferable to autoclave the final product.
   This can be done before after filling in a final packaging.
G. Quality Control
   i.The material is evaluated for purity, sterility.
   ii.The organisms are checked for acid fastness after gram staining.
   iii.Inactivation test: This is done by culturing the product on L J medium to find out any living organism.
   iv.Pathogenicity and/or contamination with pathogen.
      The cultured organisms are infected to Balb/c mice.
      None of the mice should die and all should remain healthy and gain weight. There should not be any macroscopic or microscopic lesions seen in liver, lung spleen or any other organs when animals are killed upto 8 weeks following treatment.
   v.Biochemical Test:
      The organism is subjected to following biochemical tests:
      a) Urease
      b) Tween 80 hydrolysis
      c) Niacin test
      d) Nitrate reduction test
      The organism gives negative results in urease, tween 80 hydrolysis and niacin test. It is positive by nitrate reduction test.
H. Preparation of constituents of Mycobacterium w.
   The constituents of Mycobacterium w can be prepared for the purpose of invention by:
   I. Cell disruption
   II. Solvent extration
   III. Enzymatic extraction.
The cell disruption can be done by way of sonication or use of high pressure fractionometer or by application of osmotic pressure ingredient.
The solvent extraction can be done by any organic solvent like chloroform, ethanol, methanol, acetone, phenol, isopropyl alcohol, acetic acid, urea, hexane etc.
The enzymatic extraction can be done by enzymes which can digest cell wall/membranes. They are typically proteolytic in nature. Enzyme liticase and pronase are the preferred enzymes. For the purpose of invention cell constituents of Mycobacterium w can be used alone in place of mycobacterium w organisms or it can be added to the product containing mycobacterium w.
Addition cell constituents results in improved efficacy of the product.

### Example 3. Characteristics of constituents of Mycobacterium w by HPLC analysis.

The constituents of mycobacterium w. used for the purpose of invention when subjected to HPLC analysis gives a single peak at 11 minutes. No other significant peaks are found beyond. The peak is homogenous and devoid of any notch suggesting homogeneity of material obtained

HPLC analysis was done using a waters system high performance liquid chromatography apparatus

| | |
|---|---|
| Column: | Novapak c1860A, 4µm, 3.9 x 150mm. |
| The guard column: | Novapak c 18 |
| Column Temperature: | 30° c |
| Flow rate: | 2.5 ml/min |
| Injection volume: | 25µL. |
| Mobile phase: | |
| Solvent A: HPLC grade methanol. | |
| Solvent B: HPLC grade methylene chloride | |

### Binary gradient:

The HPLC gradient initially comprised 98%(v/v) methanol (solvent B).
The gradient was increased linearly to 80%.
A and 20% B at one minute; 35% A and 65% B at 10 minutes, held for 5 seconds and then decreased over 10 seconds back to 98% A and 2% B.

### Example 4. Safety of immunomodulator;

Mycobacterium w when used in healthy animals or humans is found to be safe well tolerated and has no effect on any organ system, biochemistry or hematology including various blood cells. It is found not to cause lymphocytosis and nor change ratio of CD₄:CD₈ cells as seen with various other nonspecific immune stimulation.
The only effect seen is at injection site. It includes morphologically formation of erythema, induration ulceration and scar formation. Histologically the injection site is found to have infiltration of various kinds of lymptocytes, plasma cells, giant cells giving a histological picture of epitheloid cell granulomas.

### Example 5.Effect of immunomodulator on symptomatic HIV +ve patients ;

11 subjects who were HIV +ve and getting recurrent attacks of fever, upper respiratory tract infection, and diarrhea were given Mycobacterium w ( 5 x 10⁸) intradermally. All improved and showed no recurrence of symptoms after 2^{nd} month of treatment.

### Example 6.Effect of immunomodulator on CD₄ count in HIV 1 infected adult patients.

### a) When Immunomodulator alone is used:

In 17 HIV positive individuals who had symptoms attributed to HIV and seeking help for the same Mycobacterium w was used as a sole therapy. Mycobacterium w was administered intradermally over a deltoid region. The amount of Mycobacterium w injected was 5 x 10⁸ in a single injection. At the time of inclusion in study Mycobacterium w was given as intradermal injection over both the deltoids making a total dose of 1 x 10⁹ Mycobacterium w subsequently at the interval of a month a single intradermal injection was given over a deltoid region which included 5 x 10⁸ organisms. There was no mortality or morbidity seen during trial. All subjects tolerated the therapy well and completed the trial. Symptomatic relief was seen within two months of initiation of therapy.
All subjects were evaluated for their CD₄ count at the beginning of therapy and 5 months later. The mean pretreatment CD₄ count was 204.70 (range 430-6). All subjects showed improvement in CD₄ count. At the end of 5 months mean change in CD₄ count was 163.17 (range 8-628). In seven (41.2%) individuals increase in CD₄ count was more than 80%. Improvement in CD₄ count was less than 40% in 3 individuals (17.6%) only.
The therapy was not associated with any side effects systemically. These were a local reaction seen at the site of injection. It was in the form of erythematous reaction which was associated with induration. It progressed to ulceration at the site of injection in few which healed spontaneously leaving behind a this scar.
None of the subjects participating in a trial received any antiretroviral therapy.

### Summary of results

| | | | |
|---|---|---|---|
| A | No. of HIV +ve subjects | - | 17 |
| B | Mean base line CD₄ count | - | 204.70 |
| C | Mean post treatment CD₄ count | - | 368.93 |
| | Range | - | 850 to 32 |
| D | Change in CD₄ count | - | 163.17 |
| | | | (104.43%) |
| | Range | | 628 to 8 |
| | | | (433.33 to 4.08%) |

Details of change in CD₄ count in each individuals.(Table 1)

**Table 1**

| NO. | PRETREATMENT CD4 COUNT | POST- TREATMENT CD4 | CHANGE(NO.) | CHANGE(%) |
|---|---|---|---|---|
| 1 | 168 | 270 | 102 | 60.7 |
| 2 | 302 | 948 | 628 | 196 |
| 3 | 298 | 453 | 155 | 52 |
| 4 | 280 | 496 | 216 | 77 |
| 5 | 120 | 182 | 62 | 51.6 |
| 6 | 330 | 620 | 290 | 88 |
| 7 | 430 | 850 | 420 | 97.6 |
| 8 | 226 | 338 | 112 | 49.5 |
| 9 | 294 | 519 | 225 | 76.7 |
| 10 | 47 | 61 | 14 | 29.78 |
| 11 | 112 | 224 | 112 | 100 |
| 12 | 196 | 204 | 8 | 4.08 |
| 13 | 186 | 384 | 198 | 106.5 |
| 14 | 182 | 270 | 88 | 48.35 |
| 15 | 42 | 110 | 68 | 161.90 |
| 16 | 6 | 32 | 26 | 433.33 |
| 17 | 261 | 311 | 50 | 19.15 |
| MEAN | 204.70 | 368.93 | 163.17 | 104.43 |

The result is much better than what is achieved with use of interleukin -2 when used along with two antiretroviral drugs. It is also better than what is achieved with HAART (Highly Active AntiRetroviral Therapy) alone for the same period. It is also worth noting that all patients showed improvement of CD₄ count. Natural course of disease in abasence of antiretroviral therapy is associated with decline in CD₄ count month by month. On average 12 cells are lost per month as per ziduvadine study for symptomatic individuals published in New Eng. J. Med. In a large cohort of 2664 HIV +ve asymptomatic persons the CD4 count decline is 4.6 cells/month.

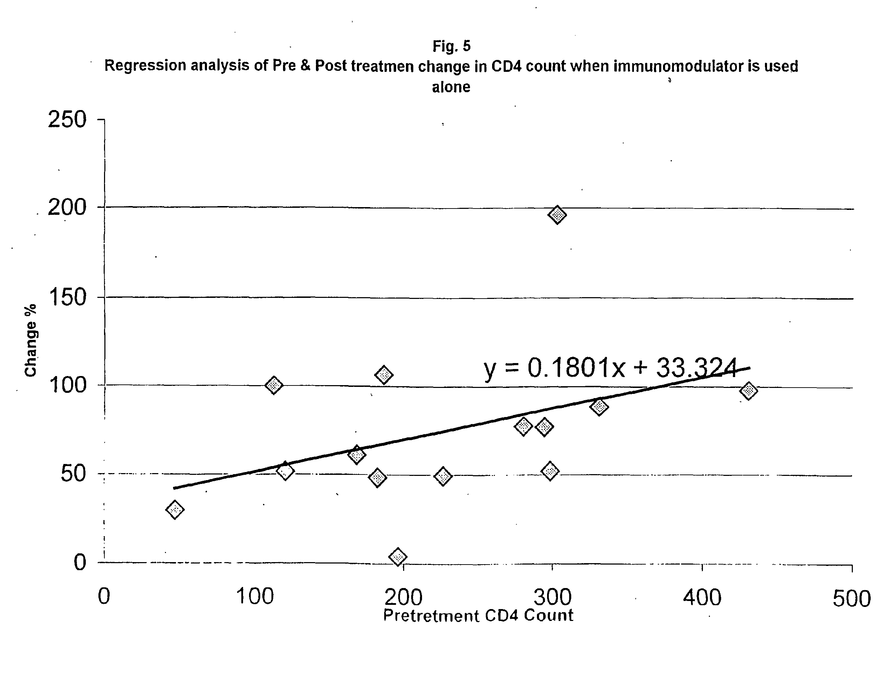

Irrespective of no. of CD₄ count at the beginning of therapy improvement in CD₄ count was seen in all individuals. The pretreatment CD₄ count ranged from 6 to 430. The regression analysis of improvement (Fig 5) suggests that improvement seen over five month period is proportionate to initial CD₄ count. Higher the count better is improvement.

### b) Two antiretroviral drugs(NRTI) + Immunomodulator

In an another set of subjects who were HIV +ve and had symptoms related to HIV Mycobacterium w was used along with two antiretroviral drugs (both NRTI). None of them had received any anti-retroviral prior to these. Mycobacterium w was administered intradermally over a deltoid region. The amount of Mycobacterium w injected was 5 x 10⁸ in a single injection. At the time of inclusion in study Mycobacterium w was given as intradermal injection over both the deltoids making a total dose of 1 x 10⁹ Mycobacterium w subsequently at the interval of a month a single intradermal injection was given over a deltoid region which included 5 x 10⁸ organisms.
There was no mortality or morbidity seen during trial. All subjects tolerated the therapy well and completed the trial. Symptomatric relief was seen within two months of initiation of therapy.
All subjects were evaluated for their CD₄ count at the beginning of therapy and 5 months later. The mean pretreatment CD₄ count was 200.99
(286 TO 96). All subjects showed improvement in CD₄ count. At the end of 5 months mean change in CD₄ count was 137.37(range 24-588)
The therapy was not associated with any side effects systemically. These were a local reaction seen at the site of injection. It was in the form of erythematous reaction which was associated with induration. It progressed to ulceration at the site of injection in few which healed spontaneously leaving behind a this scar.

None of the subjects participating in a trial received any antiretroviral therapy.

### Summary of results

| | | | |
|---|---|---|---|
| A | No. of HIV +ve subjects | - | 16 |
| B | Mean base line CD₄ count | - | 200.99 |
| C | Mean post treatment CD₄ count | - | 338.37 |
| | Range | - | 860 to 199 |
| D | Change in CD₄ count | - | 137.37 |
| | | | (68.44%) |
| | Range | | 58 to24 |
| | | | (294.15 to 16%) |

Details of change in CD₄ count in each individuals.(Table 2)

**Table 2**

| NO. | PRETREATM ENT CD4 COUNT | POST TREATMENT CD4 COUNT | CHANGE (NO.) | CHANGE(%) |
|---|---|---|---|---|
| 1 | 162 | 238 | 76 | 47 |
| 2 | 192 | 240 | 48 | 25 |
| 3 | 286 | 860 | 574 | 200 |
| 4 | 142 | 199 | 57 | 40 |
| 5 | 250 | 290 | 40 | 16 |
| 6 | 196 | 230 | 34 | 17.3 |
| 7 | 238 | 504 | 266 | 111.76 |
| 8 | 216 | 328 | 112 | 51.85 |
| 9 | 236 | 824 | 588 | 249.15 |
| 10 | 194 | 236 | 42 | 21.64 |
| 11 | 96 | 210 | 114 | 118.75 |
| 12 | 262 | 319 | 57 | 21.75 |
| 13 | 244 | 310 | 66 | 27.04 |
| 14 | 160 | 210 | 50 | 31.25 |
| 15 | 230 | 280 | 50 | 21.73 |
| 16 | 112 | 136 | 24 | 21.42 |
| Mean | 200.99 | 338.37 | 137.37 | 68.44 |

All subjects were evaluated for their CD₄ count at the beginning of therapy and 5 months later. None of the subjects showed deterioration in CD₄ count. All irrespective of pretreatment CD₄ count (Range 96 to 286) showed improvement in CD₄ count. Natural course of disease suggest minimal or no change in CD₄ when two antiretroviral drugs arc used as used in this study. Thus improvement seen in the steady is significantly much more and can not be attributed to antiretroviral therapy used in the study.

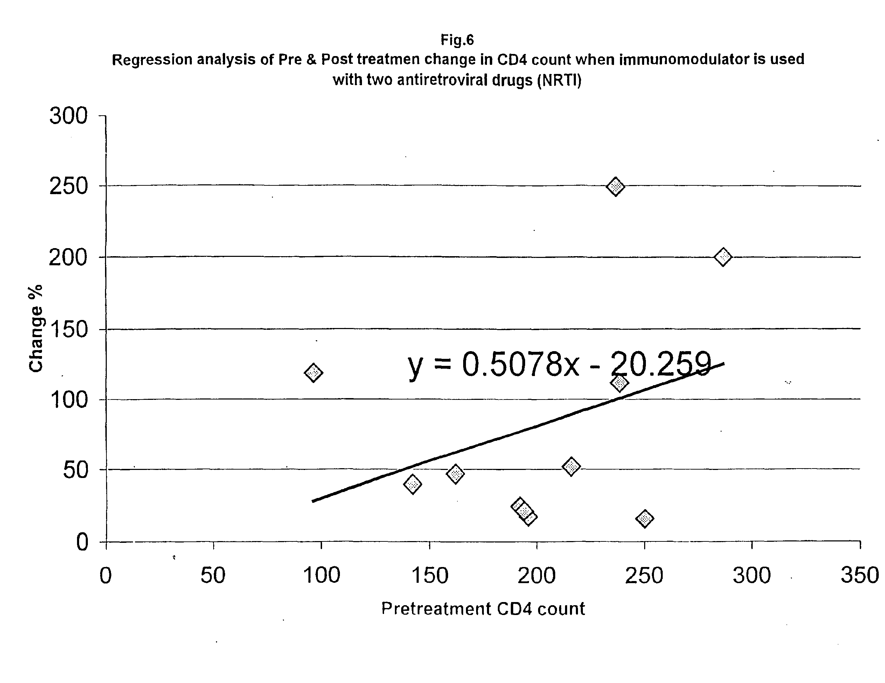

Regression analysis (Fig. 6) shows that improvement seen in CD₄ count is proportionate to the initial CD₄ count. Higher the initial count better is improvement following therapy comparison of improvement between patients receiving antiretroviral therapy (two drugs) and those not receiving therapy shows that rate of improvement is better without use of two antiretroviral drugs when initial CD₄ count is low. However when initial CD₄ count is high the rate of improvement is better when two anti-retroviral drugs are used.

### c) HAART therapy + Immunomodulator

In an another set of subjects who were HIV +ve and had symptoms related to HIV. Mycobacterium w was administered along with HAART therapy (three drugs). None of them had received any anti-retroviral prior to this. Mycobacterium w was administered intradermally over a deltoid region. The amount of Mycobacterium w injected was 5 x 10⁸ in a single injection. At the time of inclusion in study Mycobacterium w was given as intradermal injection over both the deltoids making a total dose of 1 x 10⁹ Mycobacterium w subsequently at the interval of a month a single intradermal injection was given over a deltoid region which included 5 x 10⁸ organisms.
There was no mortality or morbidity seen during trial. All subjects tolerated the therapy well and completed the trial. Symptomatic relief was seen within two months of initiation of therapy.
All subjects were evaluated for their CD₄ count at the beginning of therapy and 5 months later. The mean pretreatment CD₄ count was 213.23 (range 536-40).
All subjects showed improvement in CD₄ count. At the end of 5 months mean change in CD₄ count was 258.79 (range 40-887).
The therapy was not associated with any side effects systemically. These were a local reaction seen at the site of injection. It was in the form of erythematous reaction which was associated with induration. It progressed to ulceration at the site of injection in few which healed spontaneously leaving behind a this scar.

None of the subjects participating in a trial received any antiretroviral therapy.

### Summary of results

| | | | |
|---|---|---|---|
| A | No. of HIV +ve subjects | - | 17 |
| B | Mean base line CD₄ count | - | 213.23 |
| C | Mean post treatment CD₄ count | - | 445.58 |
| | Range | - | 1423 to 130 |
| D | Change in CD₄ count | - | 258.79 |
| | | | (155.85%) |
| | Range | | 887 to 40 |
| | | | (338.88 to 17.4%) |

Details of change in CD₄ count in each individuals.(Table 3)

**Table 3**

| NO | PRETREATMEN T CD4 COUNT | POST TREATMENT | CHANGE(NO.) | CHANCE(%) |
|---|---|---|---|---|
| 1 | 72 | 220 | 148 | 205 |
| 2 | 130 | 230 | 100 | 77 |
| 3 | 40 | 130 | 90 | 225 |
| 4 | 230 | 270 | 40 | 17.4 |
| 5 | 127 | 276 | 149 | 117 |
| 6 | 148 | 336 | 188 | 127 |
| 7 | 230 | 490 | 260 | 113.04 |
| 8 | 356 | 950 | 594 | 166.85 |
| 9 | 199 | 432 | 233 | 117.08 |
| 10 | 236 | 539 | 303 | 128.38 |
| 11 | 204 | 660 | 456 | 223.5 |
| 12 | 536 | 1423 | 887 | 165.48 |
| 13 | 108 | 269 | 161 | 149.07 |
| 14 | 92 | 198 | 106 | 115.21 |
| 15 | 203 | 582 | 379 | 186.69 |
| MEAN | 194.06 | 466.99 | 266.90 | 137.06 |
| 16 | 72 | 316 | 244 | 338.88 |
| 17 | 60 | 212 | 152 | 253.33 |
| MEAN | 66 | 264 | 198 | 296.10 |
| MEAN | 213.23 | 445.58 | 258.79 | 155.85 |

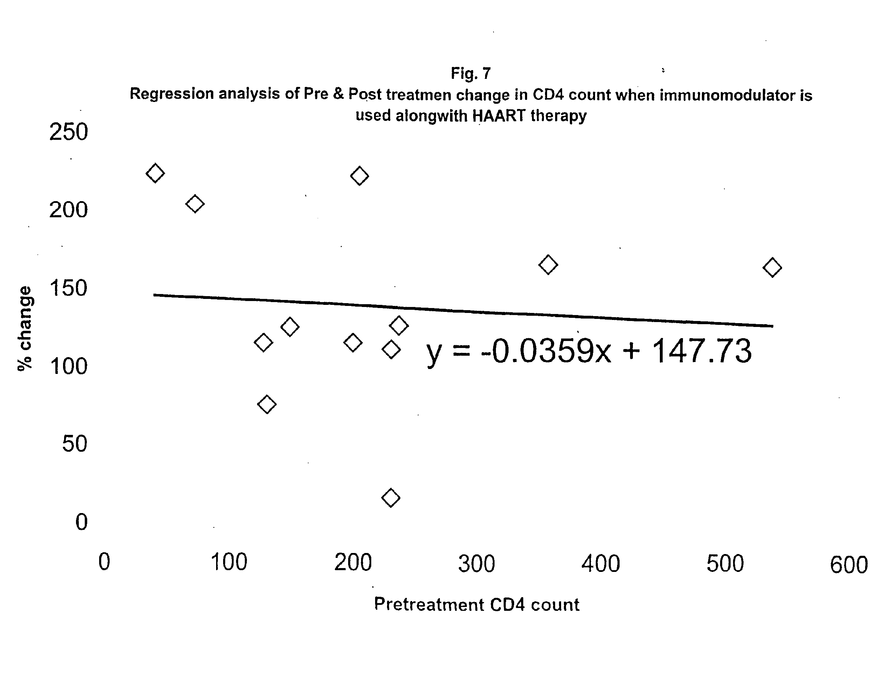

All subjects were evaluated for change in CD₄ count at the beginning of therapy and end of therapy. All patients showed significant improvement in CD₄ count. The patients no. I to 15 had NNRTI as third drug. The patient No. 16 and 17 had protease inhibitor used as third drug. The improvement was significantly more than even reported in literature. The improvement in CD₄ count was significantly more when protease inhibitor is used compared to when NNRTI is used as part of HAART therapy. Regression analysis of results (Fig.7) suggests that rate of improvement seen is more or less identical irrespective of initial CD₄ count. It was little lower when initial CD₄ count was higher compared to when it was lower.

### Example 7. Effect of immunomodulator on CD₄ count in HIV-I infected children.

Effect of Immunomodulator in children is also evaluated. Immunomodulator was given as intradermal injection of 0.1 ml every month over a deltoid region for five months. Of the five children treated with Immunomodulator alone. All showed improvement.

### CHILDREN

**TABLE : PRE AND POST Treatment CD4 COUNT**

| **No** | **PRETREATMENT** | **POST TREATMENT** | **CHANGE(NO.)** | **CHANGE(%)** |
|---|---|---|---|---|
| 28 | 506 | 1100 | 594 | 117.39 |
| 29 | 246 | 720 | 474 | 192.68 |
| 30 | 398 | 562 | 164 | 41.21 |
| 31 | 720 | 1230 | 510 | 70.83 |
| 32 | 1120 | 1460 | 340 | 30.36 |
| **MEAN** | 598 | 1014.4 | 416.4 | 90.49 |

Thus effect of Immunomodulator is not restricted to age of HIV positive patients.

### Example 8. Effect of Immunomodulator in HIV - 2 infected individuals

In another set of three subjects (HIV II positive), Immunomodulator alone was given as a therapy. It was given intradermally over a deltoid region. The amount of Immunomodulator injected was 0.1 ml in a single injection. At the time of inclusion in study Immunomodulator was given as intradermal injection over both the deltoids making a total dose of 0.2 ml subsequently at the interval of a month a single intradermal injection was given over a deltoid region.

All the three subjects showed improvement in CD₄ count.

| No. of subjects | Gender | Pre-treatement CD₄ count | Post Treatment CD₄ count | Change in CD₄ count | % |
|---|---|---|---|---|---|
| 46 | Female | 268 | 312 | 54 | 17.30 |
| 32 | Female | 324 | 402 | 78 | 19.40 |
| 49 | Male | 363 | 427 | 64 | 14.98 |

Thus effect of Immunomodulator is not found to be limited to HIV I only. Example 9. Effect of Immunomodulator in HIV +ve subjects with tuberculosis cervical lymphadenopathy not responding to five anti tuberculosis drugs.

Seven HIV +ve subjects with tuberculosis cervical lymphadenopathy not responding to five anti tuberculosis drugs were given Immunomodulator intradermally. All had initial increase in size of cervical lymph node, which became erythematous. Within 3 weeks the size of lymph nodes decreased in size and over two months lymphodenopathy healed completely.

Mycobacterium w has been used in leprosy for faster clearance of M. Leprae from lesions and improved clinical out come making it possible to release the patients from therapy (MDT, multi drug therapy) at an earlier date. It has also been found to convert lepromin negative persons to lepromin positive and there by provide immunity against leprosy. According to present invention it is found useful in management of HIV. It is seen that HIV related symptoms disappear quickly when Mycobacterium w is administered. It is also found to improve immunity in the form of CD₄ count.

It does all this in absence of any anti retroviral therapy.

However when anti-retroviral are included along with Mycobacterium w in the form of HAART therapy, response is augmented.

The lack of systemic side effects as seen with all other therapies makes it even more suitable.

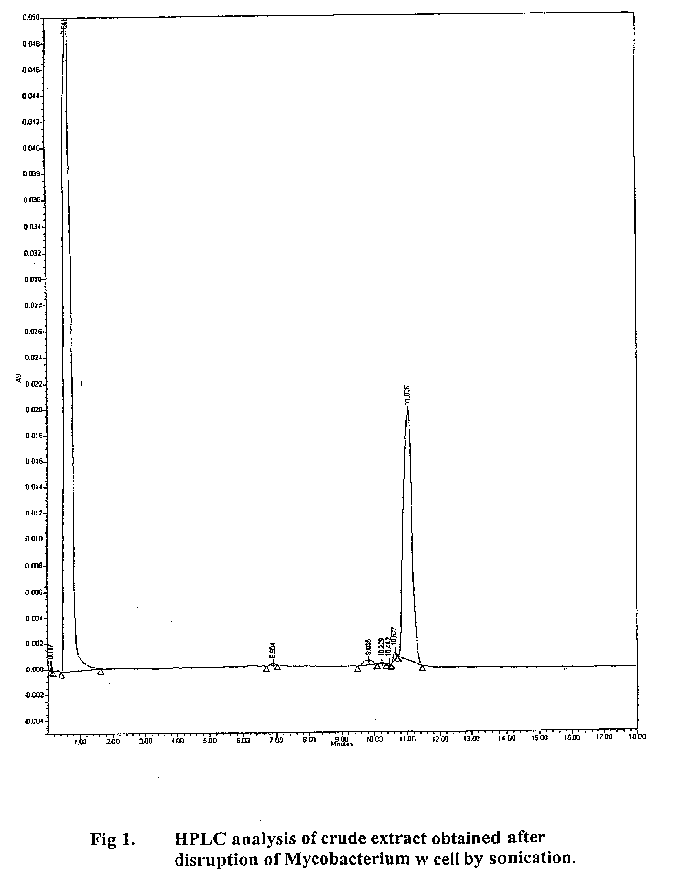

- Fig 1.: HPLC analysis of crude extract obtained after disruption of Mycobacterium w cell by sonication.

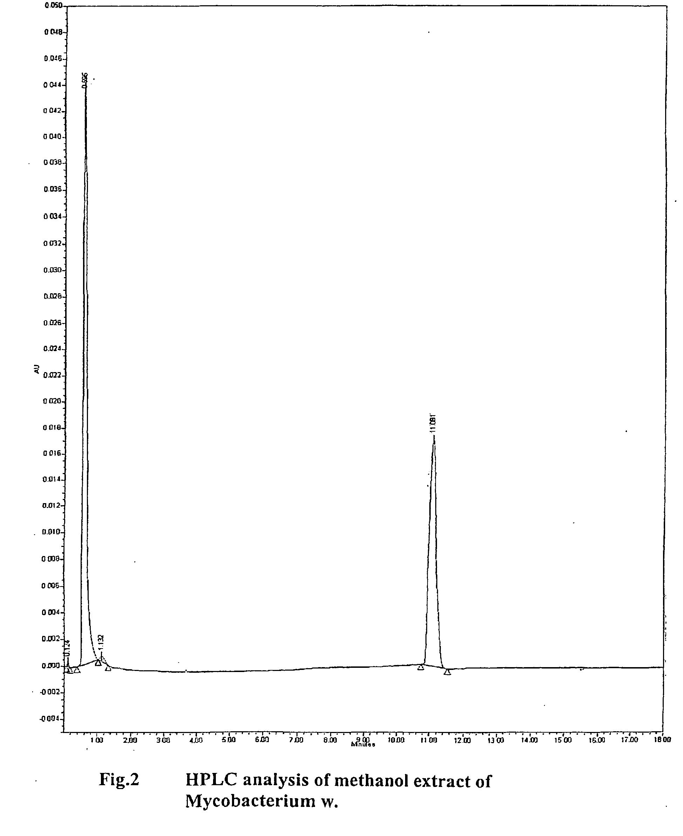

- Fig.2: HPLC analysis of methanol extract of Mycobacterium w.

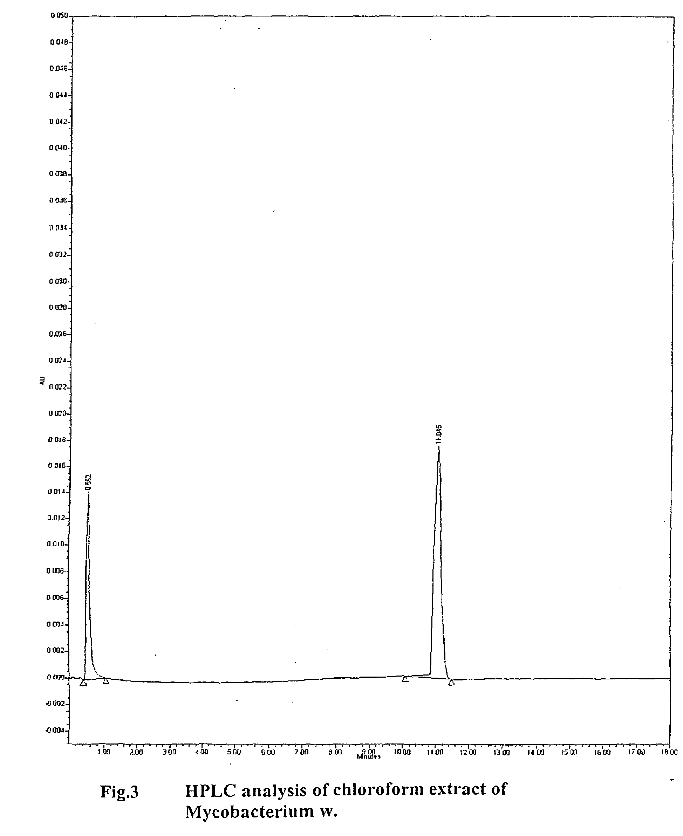

- Fig.3: HPLC analysis of chloroform extract of Mycobacterium w.

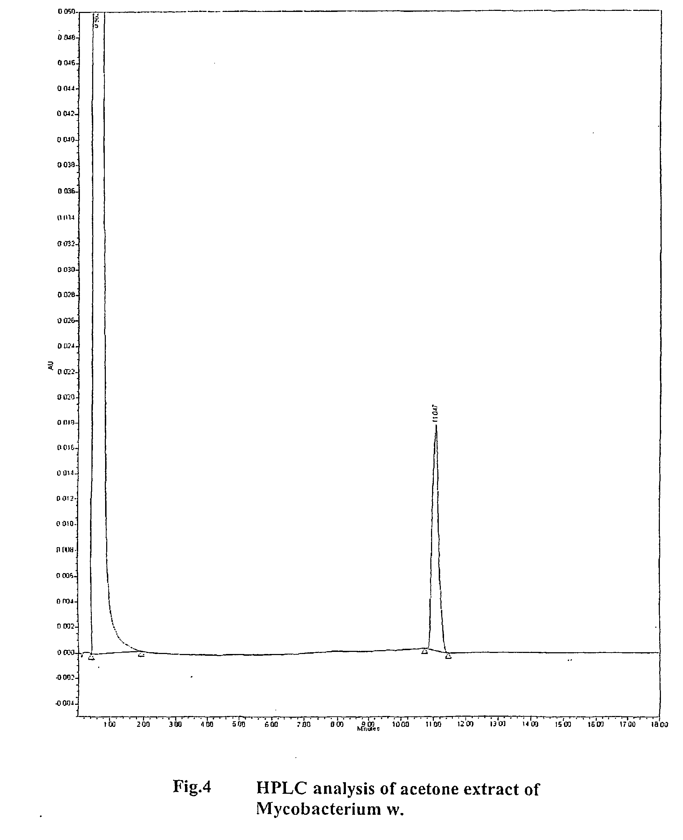

- Fig.4: HPLC analysis of acetone extract of Mycobacterium w.
- Fig.5: Regression analysis of Pre & Post treatmen change in CD4 count when immunomodulator is used alone
- Fig.6: Regression analysis of Pre & Post treatmen change in CD4 count when immunomodulator is used with two antiretroviral drugs (NRTI)
- Fig.7: Regression analysis of Pre & Post treatmen change in CD4 count when immunomodulator is used alongwith HAART therapy

## Claims

1. Use of mycobacterium w or its constituents in the preparation of a medicament for use in the management of HIV infection.

2. Use according to claim 1, wherein said medicament is for use in improving the immune function of HIV positive subjects.

3. Use according to claim 1, wherein said medicament is for use in managing opportunistic infections associated with HIV infection.

4. Use according to claim 1, wherein said medicament is for use in amelioration of symptoms associated with HIV.

5. Use according to claim 1, wherein said medicament is for use in the prophylaxis or treatment of AIDS or AIDS related complex (ARC).

6. Use according to claim 1, wherein said medicament is for use in delaying development of AIDS or AIDS related complex (ARC) in patients infected by HIV.

7. Use according to claim 1, wherein said medicament is for use in causing regression or removal, of overt symptoms of AIDS even in patients where the disease is far advanced.

8. Use according to claim 1, wherein said medicament is for use in treating patients who are suffering from HIV infection with or without AIDS and with or without tuberculosis.

9. Use according to claim 1, wherein said medicament is for use in improving CD₄+ T cell count in patients who are suffering from HIV.

10. Use according to claim 1, wherein said medicament is for use in improving CD₄+ T cell count in patients who are suffering from HIV in the absence of as well as in the presence of antiretroviral therapy.

11. The use as claimed in any of the preceding claims wherein mycobacterium w or its constituents are used alone or in combination with each other.

12. The use as claimed in claim 11 wherein the medicament further comprises adjuvants, excipients, diluents, suspending agents or preservatives.

13. The use as claimed in any of the preceding claims wherein the mycobacterium w is dead mycobacterium w.

14. The use as claimed in claim 13 wherein the mycobacterium w is killed by physical methods like heat or radiation by heat in the form of autoclaving.

15. The use as claimed in claim 14 wherein the mycobacterium is killed by heat in the form of autoclaving.

16. The use as claimed in any one of claims 1 to 10 wherein mycobacterium w constituents are used and are obtained by sonication.

17. The use as claimed in any one of claims 1 to 10 wherein mycobacterium w constituents are used and are obtained by a high pressure cell fractionator.

18. The use as claimed in any one of claims 1 to 10 wherein mycobacterium w constituents are used and are obtained by osmatic pressure ingredient.

19. The use as claimed in any one of claims 1 to 10 wherein mycobacterium w constituents are used and are obtained by extraction.

20. The use as claimed in claim 19 wherein the mycobacterium w constituents are extracted by organic solvents.

21. The use as claimed in claim 20 wherein the organic solvents are selected from chloroform, ethanol, methanol, acetone, phenol, isopropyl alcohol, acetic acid, urea and hexane.

22. The use as claimed in any one of claims 1 to 10 wherein mycobacterium w constituents are used and are obtained by enzymatic treatment.

23. The use as claimed in claim 22 wherein the mycobacterium w is obtained by use of enzyme lyticase and/or pronase.

24. The use as claimed in any one of claims 16 to 23 wherein the mycobacterium w constituents have a molecular weight of more than 12,000 daltons.

25. The use as claimed in any one of claims 16 to 23 wherein the mycobacterium w constituents are water insoluble.

26. The use as claimed in any one of claims 1 to 10 wherein the medicament is in a unit dosage form comprising equal to or more than 1 x 10⁵ mycobacterium w.

27. The use as claimed in any one of claims 1 to 10 wherein the medicament is in a unit dosage form comprising equal to or more than 1 x 10⁷ mycobacterium w.

28. The use as claimed in any one of claims 1 to 10 wherein the medicament is in a unit dosage form comprising between 1 x 10⁸ and 1 x 10¹⁰ mycobacterium w.

29. The use as claimed in any one of claims 1 to 10 wherein the mycobacterium w is urease negative, does not hydrolyse polyoxyethylene sorbitan monooleate, does not produce niacin and provides a strong positive response to nitrate reduction test.

## Patentansprüche

1. Verwendung von Mycobacterium w oder seinen Bestandteilen bei der Zubereitung eines Medikaments zur Verwendung in der Behandlung einer HIV Infektion.

2. Verwendung gemäß Anspruch 1, worin das Medikament für den Einsatz bei der Verbesserung der Immunfunktion von HIVpositiven Individuen bestimmt ist.

3. Verwendung gemäß Anspruch 1, worin das Medikament für den Einsatz in der Behandlung von mit einer HIV Infektion verbundenen opportunistischen Infektionen bestimmt ist.

4. Verwendung gemäß Anspruch 1, worin das Medikament für den Einsatz zur Besserung von mit einer HIV Infektion verbundenen Symptomen bestimmt ist.

5. Verwendung gemäß Anspruch 1, worin das Medikament für den Einsatz in der Prophylaxe oder der Behandlung von AIDS oder AIDS-related Complex (ARC) bestimmt ist.

6. Verwendung gemäß Anspruch 1, worin das Medikament für den Einsatz zur Verzögerung der Entwicklung von AIDS oder AIDS-related Complex (ARC) bei mit HIV infizierten Patienten bestimmt ist.

7. Verwendung gemäß Anspruch 1, worin das Medikament für den Einsatz zum Herbeiführen einer Rückbildung oder der Beseitigung offenkundiger Symptome von AIDS sogar bei Patienten, bei denen die Krankheit weit fortgeschritten ist, bestimmt ist.

8. Verwendung gemäß Anspruch 1, worin das Medikament für den Einsatz bei der Behandlung von Patienten, die an einer HIV Infektion mit oder ohne AIDS und mit oder ohne Tuberkulose leiden, bestimmt ist.

9. Verwendung gemäß Anspruch 1, worin das Medikament für den Einsatz zur Verbesserung der Anzahl von CD₄+ T-Zellen bei Patienten, die an HIV leiden, bestimmt ist.

10. Verwendung gemäß Anspruch 1, worin das Medikament für den Einsatz zur Verbesserung der Anzahl von CD₄+ T-Zellen bei Patienten, die an HIV leiden, bestimmt ist, sowohl ohne eine antiretrovirale Therapie als auch in Verbindung mit einer antiretroviralen Therapie.

11. Verwendung gemäß irgendeinem der voranstehenden Ansprüche, worin Mycobacterium w oder seine Bestandteile einzeln oder zusammen eingesetzt werden.

12. Verwendung gemäß Anspruch 11, worin das Medikament außerdem Hilfsstoffe, Vehikel bzw. sonstige Bestandteile, Verdünnungsmittel, Suspendiermittel oder Konservierungsmittel enthält.

13. Verwendung gemäß irgendeinem der voranstehenden Ansprüche, worin das Mycobacterium w totes Mycobacterium w ist.

14. Verwendung gemäß Anspruch 13, worin das Mycobacterium w durch physikalische Methoden wie Hitze oder Strahlung durch Hitze in Form von Autoklavierung abgetötet wird.

15. Verwendung gemäß Anspruch 14, worin das Mycobacterium durch Hitze in Form von Autoklavierung abgetötet wird.

16. Verwendung gemäß irgendeinem der Ansprüche 1 bis 10, worin Bestandteile von Mycobacterium w eingesetzt werden und durch Ultraschallbehandlung erhalten werden.

17. Verwendung gemäß irgendeinem der Ansprüche 1 bis 10, worin Bestandteile von Mycobacterium w eingesetzt werden und mittels eines Hochdruck-Zellfraktionators erhalten werden.

18. Verwendung gemäß irgendeinem der Ansprüche 1 bis 10, worin Bestandteile von Mycobacterium w eingesetzt werden und durch eine Zutat des osmotischen Drucks erhalten werden.

19. Verwendung gemäß irgendeinem der Ansprüche 1 bis 10, worin Bestandteile von Mycobacterium w eingesetzt werden und durch Extraktion erhalten werden.

20. Verwendung gemäß Anspruch 19, worin die Bestandteile von Mycobacterium w mit organischen Lösungsmitteln extrahiert werden.

21. Verwendung gemäß Anspruch 20, worin die organischen Lösungsmittel ausgewählt sind aus Chloroform, Ethanol, Methanol, Aceton, Phenol, Isopropanol, Essigsäure, Harnstoff und Hexan.

22. Verwendung gemäß irgendeinem der Ansprüche 1 bis 10, worin Bestandteile von Mycobacterium w eingesetzt werden und durch enzymatische Behandlung erhalten werden.

23. Verwendung gemäß Anspruch 22, worin das Mycobacterium w durch den Einsatz der Enzyme Lyticase und/oder Pronase erhalten wird.

24. Verwendung gemäß irgendeinem der Ansprüche 16 bis 23, worin die Bestandteile von Mycobacterium w ein Molekulargewicht von mehr als 12.000 Dalton aufweisen.

25. Verwendung gemäß irgendeinem der Ansprüche 16 bis 23, worin die Bestandteile von Mycobacterium w wasserunlöslich sind.

26. Verwendung gemäß irgendeinem der Ansprüche 1 bis 10, worin das Medikament in einer Verabreichungsform vorliegt, die eine Dosis von 1 x 10⁵ oder mehr Mycobacterium w enthält.

27. Verwendung gemäß irgendeinem der Ansprüche 1 bis 10, worin das Medikament in einer Verabreichungsform vorliegt, die eine Dosis von 1 x 10⁷ oder mehr Mycobacterium w enthält.

28. Verwendung gemäß irgendeinem der Ansprüche 1 bis 10, worin das Medikament in einer Verabreichungsform vorliegt, die eine Dosis von zwischen 1 x 10⁸ und 1 x 10¹⁰ Mycobacterium w enthält.

29. Verwendung gemäß irgendeinem der Ansprüche 1 bis 10, worin das Mycobacterium w Urease-negativ ist, Polyoxyethylensorbitanmonooleat nicht hydrolysiert, kein Niacin produziert und eine stark positive Reaktion im Nitratreduktionstest zeigt.

## Revendications

1. Utilisation de mycobactérium w ou de ses constituants dans la préparation d'un médicament qui peut être utilisé pour prendre en charge l'infection par le VIH.

2. Utilisation selon la revendication 1, dans laquelle ledit médicament peut être utilisé pour améliorer la fonction immune des sujets séropositifs au VIH.

3. Utilisation selon la revendication 1, dans laquelle ledit médicament peut être utilisé pour prendre en charge les infections opportunistes associées à l'infection par le VIH.

4. Utilisation selon la revendication 1, dans laquelle ledit médicament peut être utilisé pour atténuer les symptômes associés au VIH.

5. Utilisation selon la revendication 1, dans laquelle ledit médicament peut être utilisé en prophylaxie ou pour traiter le SIDA ou le complexe lié au SIDA (ARC).

6. Utilisation selon la revendication 1, dans laquelle ledit médicament peut être utilisé pour retarder le développement du SIDA ou du complexe lié au SIDA (ARC) chez les patients infectés par le VIH.

7. Utilisation selon la revendication 1, dans laquelle ledit médicament peut être utilisé pour induire la régression ou l'élimination des symptômes patents du SIDA, même chez les patients chez qui la maladie est très avancée.

8. Utilisation selon la revendication 1, dans laquelle ledit médicament peut être utilisé pour traiter les patients qui souffrent d'une infection par le VIH, avec ou sans avoir déclaré le SIDA, et avec ou sans tuberculose.

9. Utilisation selon la revendication 1, dans laquelle ledit médicament peut être utilisé pour améliorer le nombre de lymphocytes T CD₄+ chez les patients qui souffrent du VIH.

10. Utilisation selon la revendication 1, dans laquelle ledit médicament peut être utilisé pour améliorer le nombre de lymphocytes T CD₄+ chez les patients qui souffrent du VIH, en l'absence ou en présence d'une thérapie antirétrovirale.

11. Utilisation selon l'une quelconque des revendications précédentes, dans laquelle mycobacterium w ou ses constituants sont utilisés seuls ou en combinaison les uns avec les autres.

12. Utilisation selon la revendication 11, dans laquelle ledit médicament comprend, en outre, des adjuvants, des excipients, des diluants, des agents de mise en suspension ou des conservateurs.

13. Utilisation selon l'une quelconque des revendications précédentes, dans laquelle mycobacterium w est un mycobacterium w mort.

14. Utilisation selon la revendication 13, dans laquelle mycobacterium w est tué par des procédés physiques comme la chaleur ou un rayonnement chaud par autoclavage.

15. Utilisation selon la revendication 14, dans laquelle mycobacterium w est tué par la chaleur par autoclavage.

16. Utilisation selon l'une quelconque des revendications 1 à 10, dans laquelle les constituants de mycobactérium w sont utilisés et sont obtenus par sonication.

17. Utilisation selon l'une quelconque des revendications 1 à 10, dans laquelle les constituants de mycobactérium w sont utilisés et sont obtenus par fractionnement cellulaire à haute pression.

18. Utilisation selon l'une quelconque des revendications 1 à 10, dans laquelle les constituants de mycobactérium w sont utilisés et sont obtenus par un ingrédient de pression osmotique.

19. Utilisation selon l'une quelconque des revendications 1 à 10, dans laquelle les constituants de mycobactérium w sont utilisés et sont obtenus par extraction.

20. Utilisation selon la revendication 19, dans laquelle les constituants de mycobactérium w sont extraits par des solvants organiques.

21. Utilisation selon la revendication 20, dans laquelle les solvants sont choisis parmi le chloroforme, l'éthanol, le méthanol, l'acétone, le phénol, l'alcool isopropylique, l'acide acétique, l'urée et l'hexane.

22. Utilisation selon l'une quelconque des revendications 1 à 10, dans-laquelle les constituants de mycobactérium w sont utilisés et sont obtenus par traitement enzymatique.

23. Utilisation selon la revendication 22, dans laquelle mycobactérium w est obtenu en utilisant une enzyme lyticase et/ou pronase.

24. Utilisation selon l'une quelconque des revendications 16 à 23, dans laquelle les constituants de mycobactérium w ont un poids moléculaire supérieur à 12 000 daltons.

25. Utilisation selon l'une quelconque des revendications 16 à 23, dans laquelle les constituants de mycobactérium w sont insolubles dans l'eau.

26. Utilisation selon l'une quelconque des revendications 1 à 10, dans laquelle le médicament est sous une forme de dosage unitaire comprenant une concentration de mycobacterium w égale ou supérieure à 1 x 10⁵.

27. Utilisation selon l'une quelconque des revendications 1 à 10, dans laquelle le médicament est sous une forme de dosage unitaire comprenant une concentration de mycobacterium w égale ou supérieure à 1 x 10⁷.

28. Utilisation selon l'une quelconque des revendications 1 à 10, dans laquelle le médicament est sous une forme de dosage unitaire comprenant une concentration de mycobacterium w entre 1 x 10⁸ et 1 x 10¹⁰.

29. Utilisation selon l'une quelconque des revendications 1 à 10, dans laquelle mycobacterium w est négatif à l'uréase, n'hydrolyse pas le monooléate de polyoxyéthylène sorbitan, ne produit pas de niacine et fournit une réponse positive forte au test de réduction au nitrate.
